# EUROPÄISCHE PATENTSCHRIFT

(11) **EP 1 073 634 B1**
(45) Veröffentlichungstag und Bekanntmachung des Hinweises auf die Patenterteilung: **31.08.2005**
(21) Anmeldenummer: 99923421.4
(22) Anmeldetag: 16.04.1999
(51) Int. Cl.: C07D 207/12, A61K 31/40, C07B 43/06, C07B 35/02

(54) **VERFAHREN ZUR HERSTELLUNG VON ENANTIOMERENREINEM N-METHYL- N- (1S)- 1-PHENYL- 2-((3S)-3- HYDROXYPYRROLIDIN- 1-YL)ETHYL]- 2,2- DIPHENYLACETAMID**
METHOD FOR PRODUCING ENANTIOMERICALLY PURE N-METHYL-N- (1S)-1-PHENYL- 2-((3S)- 3-HYDROXYPYRROLIDINE- 1-YL)ETHYL]- 2,2-DIPHENYL ACETAMIDE
PROCEDE DE PRODUCTION DE N-METHYL-N-((1S)-1-PHENYL-2-((3S)-3-HYDROXYPYRROLIDIN-1-YL)ETHYL)-2,2-DIPHENYLACETAMIDE ENANTIOMERIQUEMENT PUR

(30) Priorität: 20.04.1998 DE 19817393; 20.06.1998 DE 19827633
(43) Veröffentlichungstag der Anmeldung: 07.02.2001
(73) Patentinhaber: Merck Patent GmbH, 64293 Darmstadt (DE)
(72) Erfinder: BATHE, Andreas, D-64283 Darmstadt (DE); HELFERT, Bernd, D-64372 Ober-Ramstadt (DE); ACKERMANN, Karl-August, D-64372 Ober-Ramstadt (DE); GOTTSCHLICH, Rudolf, D-64354 Reinheim (DE); STEIN, Ingeborg, D-63110 Rodgau (DE); BUDAK, Jens, D-64287 Darmstadt (DE)
(86) Internationale Anmeldenummer: PCT/EP1999/002574
(87) Internationale Veröffentlichungsnummer: WO 1999/054298

(56) Entgegenhaltungen:
- DE-A- 4 034 785
- DE-A- 4 215 213
- DE-A- 19 523 502
- DE-A- 19 647 538
- GOTTSCHLICH R ET AL: "EMD 61 753 AS A FAVOURABLE REPRESENTATIVE OF STRUCTURALLY NOVEL ARYLACETAMIDO-TYPE K OPIATE RECEPTOR AGONISTS" BIOORGANIC & MEDICINAL CHEMISTRY LETTERS, Bd. 4, Nr. 5, 1. Januar 1994 (1994-01-01), Seiten 677-682, XP000602924 ISSN: 0960-894X
- BARBER A ET AL: "A PHARMACOLOGICAL PROFILE OF THE NOVEL, PERIPHERALLY-SELECTIVE KAPPA-OPIOID RECEPTOR AGONIST, EMD 61753" BRITISH JOURNAL OF PHARMACOLOGY, Bd. 113, Nr. 4, 1. Dezember 1994 (1994-12-01), Seiten 1317-1327, XP000196255 ISSN: 0007-1188 in der Anmeldung erwähnt
- PINNA G. ET AL: 'Synthesis and kappa binding affinity of 1-(pyrrolidin-1-ylmethyl)-2-(N-methyl)-4-[( 3,4-dichloro)phenyl]-1,2,3,4-tetrahydroisoq uinolin-3(2H)-ones' EUROPEAN JOURNAL OF MEDICINAL CHEMISTRY, EDITIONS SCIENTIFIQUE Bd. 30, Nr. 6, 1995, ELSEVIER, PARIS, FR, Seiten 515 - 520

## Beschreibung

Die Erfindung betrifft ein neues Verfahren zur wahlweisen Herstellung von N-Methyl-N-[(1S)-1-phenyl-2-((3S)-3-hydroxypyrrolidin-1-yl)-ethyl]-2,2-diphenylacetamid oder N-Methyl-N-[(1R)-1-phenyl-2-((3R)-3-hydroxypyrrolidin-1-yl)-ethyl]-2,2-diphenylacetamid.

Wie von Barber et al. (B. J. Pharmacol. (1994), **113**, 1317-1327) beschrieben, besitzen sowohl die Verbindung N-Methyl-N-[(1S)-1-phenyl-2-((3S)-3-hydroxypyrrolidin-1-yl)-ethyl]-2,2-diphenylacetamid, als auch ihre physiologisch verträglichen Salze wertvolle pharmakologische Eigenschaften wie eine anaigetische, antiinflammatorische und aquaretische Wirkung, so daß sie besonders zur Herstellung von Arzneimitteln geeignet sind.

Es wurde, wie in der Patentanmeldung DE 1 95 23 502 bzw. EP 752 246 beschrieben, gefunden, daß diese Verbindung eine besonders wirksame Verbindung ist, die sich als Arzneimittel zur Behandlung entzündlicher Darmerkrankungen in ganz besonderer Weise eignet. Insbesondere ist diese Verbindung bei dieser Indikation einsetzbar und wirksam, da sie gleichzeitig die mit dieser Erkrankung verbundenen Schmerzen lindert und im akuten Fall eines durch die entzündliche Darmerkrankung drohenden bzw. hervorgerufenen Darmverschlusses die Motorik des Darms wieder normalisiert oder wieder in Gang setzt, ohne spürbare Nebenwirkungen hervorzurufen. Außerdem kann die Verbindung bei nicht-entzündlichen Darmerkrankungen wie IBS (Irritable Bowel Syndrome) eingesetzt werden.

In den Patentanmeldungen DE 42 15 213 A1 bzw. EP 0 569 802 A1 ist die Herstellung von N-Methyl-N-[(1S)-1-phenyl-2-((3S)-3-hydroxypyrrolidin-1-yl)-ethyl]-2,2-diphenylacetamid durch Umsetzung von (1S)-[1-N-Methyl-amino-1-phenyl-2-(3S)-3-hydroxypyrrolidino)]ethan mit Diphenylacetylchlorid beschrieben. Wie in DE 42 15 213 beschrieben, ist die Ausgangsverbindung (1S)-[1-N-Methyl-amino-1-phenyl-2-((3S)-3-hydroxy-pyrrolidino)]ethan herstellbar, indem (1S)-1-Amino-1-phenyl-2-chlorethan mit (3S)-3-Hydroxypyrrolidin umgesetzt und anschließend mit Methyljodid methyliert wird. Probleme dieser Herstellungsmethode bestehen jedoch in der Löslichkeit der Ausgangsprodukte und darin, daß im Anschluß an die Synthese das erhaltene, durch Nebenprodukte verunreinigte racemische Produktgemisch aufwendig aufgetrennt werden muß. Das bisher bekannte Verfahren zur Herstellung von N-Methyl-N-[(1S)-1-phenyl-2-((3S)-3-hydroxypyrrolidin-1-yl)-ethyl]-2,2-diphenylacetamid ist daher aufwendig und teuer und führt zu geringen Ausbeuten bezogen auf die eingesetzten Ausgangsverbindungen.

In Bioorg. Med. Chem. Letters, Bd. 4, Nr. 5, 1994, Seiten 677-682 ist die Herstellung von N-Methyl-N-[(1S)-1-phenyl-2-((3S)-3-hydroxypyrrolidin-1-yl)-ethan]-amin durch Reduktion von (2S)-N-Formyl-2-phenylglycin-N,N-[(3S)-3-hydroxy-tetramethylenamid] beschrieben. Eine analoge Reaktion wird in DE 40 34 785 A1 beschrieben.

Aufgabe der vorliegenden Erfindung war es daher, ein in einfacher Weise durchführbares und preiswertes Verfahren zur Herstellung von N-Methyl-N-[(1S)-1-phenyl-2-((3S)-3-hydroxypyrrolidin-1-yl)-ethyl]-2,2-diphenylacetamid bzw. bei Einsatz der enantiomeren Edukte von N-Methyl-N-[(1R)-1-phenyl-2-((3R)-3-hydroxypyrrolidin-1-yl)-ethyl]-2,2-diphenylacetamid zur Verfügung zu stellen, das von preiswerten, gut löslichen Ausgangsprodukten ausgeht, die zu einem möglichst enantiomerenreinen Produkt führen, das sich dann anschließend in einfacher Weise isolieren und reinigen läßt.

Die Lösung der Aufgabe erfolgt durch ein Verfahren gemäß Anspruch 1, wobei entweder die Verbindung N-Methyl-N-[(1S)-1-phenyl-2-((3S)-3-hydroxypyrrolidin-1-yl)-ethan]-amin als Zwischenprodukt zur Herstellung von N-Methyl-N-[(1S)-1-phenyl-2-((3S)-3-hydroxypyrrolidin-1-yl)ethyl]-2,2-diphenylacetamid verwendet wird oder N-Methyl-N-[(1R)-1phenyl-2-((3R)-3-hydroxypyrrolidin-1-yl)-ethan]-amin als Zwischenprodukt zur Herstellung von N-Methyl-N-[(1R)-1-phenyl-2-((3R)-3-hydroxypyrrolidin-1-yl)ethyl]-2,2-diphenylacetamid.

Es wurde gefunden, daß sich Verbindungen der Formel (III) 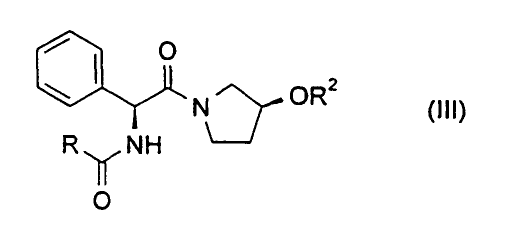 worin R und R² die folgenden Bedeutungen haben,
- R: OR¹ oder SR¹,
- R¹: A, Aryl, Heteroaryl, Si(R³)₃ oder COR³,
- R²: H, A, Aryl, Heteroaryl sowie Si(R³)₃ oder COR³,
- R³: H, A, Aryl oder Heteroaryl,
- A: geradkettiger oder verzweigter Alkylrest mit 1 bis 6 C-Atomen,
in hohen Ausbeuten und enantiomerenrein darstellen lassen, indem, je nach gewünschtem Endprodukt, (3S)-3-Hydroxypyrrolidine oder (3R)-3-Hydroxypyrrolidine der Formel (II) 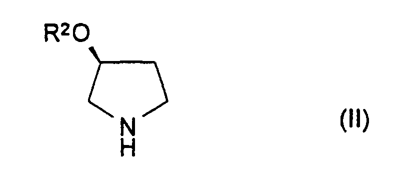 worin
- R²: H, A, Aryl, Heteroaryl sowie Si(R³)₃ oder COR³ und
- R³: H, A, Aryl oder Heteroaryl
bedeuten, oder deren Salze, gebildet mit HCl, HBr, HI, H₂SO₄, H₃PO₄ oder geeigneten organischen Säuren,
mit entsprechenden (S)- oder (R)-enantiomeren Formen von N-substituierten Phenylglycinen der Formel (I) 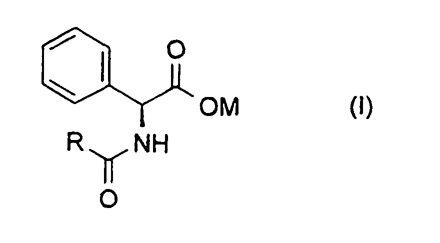 worin
- R: OR¹ oder SR¹,
- R¹: A, Aryl, Heteroaryl, Si(R³)₃ oder COR³,
- R³: H, A, Aryl oder Heteroaryl,
- M: H oder ein Kation aus der Gruppe Alkali, Erdalkali, Ammonium oder Alkylammonium
bedeuten,
amidisch gekuppelt werden.

Alkyl hat 1 bis 6, vorzugsweise 1, 2, 3 oder 4 C-Atome. Alkyl bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Aryl bedeutet vorzugsweise unsubstituiertes Phenyl oder ein- oder zweifach durch Hal, OA oder Alkyl substituiertes Phenyl, fermer z.B. Biphenyl oder Naphthyl.

Heteroaryl bedeutet vorzugsweise z.B. Furanyl, Thiophenyl, Pyridinyl, Pyrrolyl oder Thiazolyl.

Si(R³)₃ bedeutet vorzugsweise z.B. Si(CH₃)₃.

COR³ bedeutet vorzugsweise z.B. Acetyl oder Benzoyl.

R bedeutet vorzugsweise insbesondere z.B. Methoxy oder Ethoxy.

R¹ bedeutet insbesondere z.B. Methyl, Ethyl, Propyl, Butyl, Phenyl, Si(CH₃)₃ oder Acetyl.

R² bedeutet insbesondere z.B. H, tert.-Butyl, Si(CH₃)₃, Acetyl, Benzyl oder Benzoyl, ganz besonders bevorzugt ist H.

Die hergestellten Amide der Formel (III) lassen sich in einfacher Weise reduktiv, gegebenenfalls durch Abspaltung der Schutzgruppe der Hydroxylgruppe des Pyrrolidins in N-Methyl-N-[(1S)-1-phenyl-2-((3S)-3-hydroxypyrrolidin-1-yl)-ethan]-amin oder N-Methyl-N-[(1R)-1-phenyl-2-((3R)-3-hydroxypyrrolidin-1-yl)-ethan]-amin der Formel (IV) überführen.

Durch Umsetzung mit aktivierten Carbonsäuren der Formel (V) 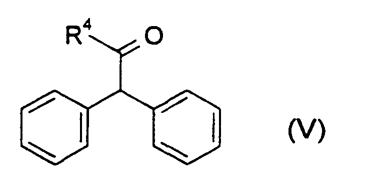 worin
- R⁴: F, Cl, Br, I, OA oder O-CO-A
bedeutet,
lassen sich aus den freien Basen der Verbindungen der Formel (IV) 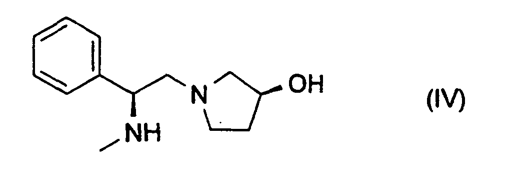 oder aus ihren Salzen, gebildet mit HCl, HBr, HI, H₂SO₄, H₃PO₄ oder geeigneten organischen Säuren die enantiomeren Verbindungen der Formel (VI) 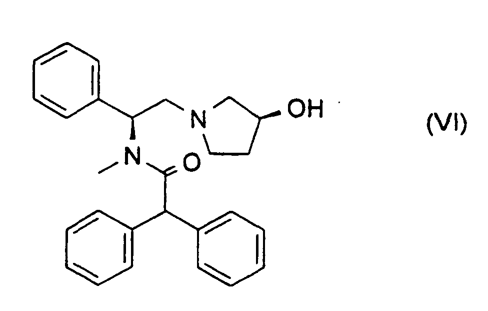 in reiner Form herstellen. Vorzugsweise werden diese Verbindungen als Hydrochloride hergestellt, wobei es sich bei der Verbindung N-Methyl-N-[(1S)-1-phenyl-2-((3S)-3-hydroxypyrrolidin-1-yl)-ethyl]-2,2-diphenylacetamid um die bekannte Form EMD 61753 handelt; aber auch die entsprechenden Salze mit den übrigen oben genannten Säuren sind analog herstellbar.

Insbesondere ist auf diese Weise durch die letzte Umsetzung mit Diphenylessigsäurechlorid N-Methyl-N-[(1S)-1-phenyl-2-((3S)-3-hydroxypyrrolidin-1-yl)-ethyl]-2,2-diphenylacetamid, herstellbar.

Die als Zwischenprodukt synthetisierten Verbindungen der Formel (IV) lassen sich allgemein durch Umsetzung von Verbindungen der Formel (I) mit solchen der Formel (II) gewinnen. Vorzugsweise werden in dieser Reaktion Verbindungen der Formel (I) verwendet, in denen R die Bedeutung OR¹ mit R¹ A, Aryl, Heteroaryl, Si(R³)₃ oder COR² und R² H, Alkyl, Aryl oder Heteroalkyl, mit den oben angegebenen bevorzugten Bedeutungen, besitzt. Überraschenderweise werden im Gegensatz zur Verwendung der entsprechenden Formylverbindung enantiomerenreine Reaktionsprodukte der Formel (III) erhalten. Auf diese Weise kann vorteilhafterweise die Auftrennung des Racemats entfallen.

Die Umsetzung der Verbindungen (I) und (II) kann in einem beliebigen aprotischen Lösungsmittel erfolgen. Besonders geeignet sind polare aprotische Lösungsmittel aus der Gruppe Diethylether, Petrolether, Aceton, Nitrobenzol, Dimethylformamid, Dimethylsulfoxid oder andere entsprechende Lösungsmittel. Hierbei werden die Edukte in so viel Lösungsmittel aufgenommen, daß eine 10 bis 30 prozentige Lösung erhalten wird. Bevorzugt wird die Reaktion in Tetrahydrofuran als Lösungsmittel durchgerührt.

Die Reaktionen der Verbindungen (I) und (II) erfolgen unter geeigneten Bedingungen bei Temperaturen zwischen 0 bis 50 °C. Besonders gute Ergebnisse werden jedoch bei Raumtemperaturen zwischen 20 und 30°C und bei Normaldruck erzielt.

Zur Aktivierung der Edukte ist die Gegenwart eines Hilfsreagenzes erforderlich. Dieses können Hilfsmittel sein, die auch als Peptidkupplungsreagenzien verwendet werden. Geeignet sind Verbindungen wie beispielsweise Phosphoroxytrichlorid, Phosphorhalogenide der Wertigkeit III und V, Phosgen, Dicyclohexylcarbodiimid, Tributylammoniumsalz des Pyridins, Phenyldichlorphosphat, 2-Chlor-1,2,3-trinitrobenzol, Phosphorsäureester, Chlorsulfonylisocyanat, CH₃SO₂Cl-(C₂H₅)₃N, (C₆H₅)₃P-CCl₄-(C₂H₅)₃N, N,N'-Carbonyldiimidazol,, N-(Alkylcarbonyl)-imidazole, Säureanhydride oder Säurechloride und insbesondere Alkylchlorformiate, wie Chlorameisensäureethylester. Andere geeignete Hilfsreagenzien sind in verschiedenen Fachbüchern beschrieben, wie z. B. in C. Ferri "Reaktionen der organischen Synthese"; R. C. Larock "Comprehensive Organic Transformations; A Guide to Functional Group Preparations", Verlag Chemie, 1989.

Weiterhin ist die Gegenwart einer Base erforderlich. Geeignete sind ebenfalls aus den oben genannten Fachbüchern zu entnehmen. Solche Basen sind beispielsweise tertiäre Amine, wie z. B. Triethylamin. Es können aber auch anorganische Basen hinzugefügt werden. Als anorganische Basen sind insbesondere Carbonate geeignet. Bei Verwendung der Alkalihydroxide, wie NaOH oder KOH ist besonders auf eine genaue Dosierung zu achten, da es sonst zu unerwünschten Nebenreaktionen kommt. Zur Vereinfachung der Aufarbeitung ist es aber auch möglich, das Hydroxypyrrolidin im Überschuß einzusetzen, so daß es selbst als Base wirkt.

Die Aufarbeitung des erhaltenen Reaktionsprodukts (III) kann nach dem Abfiltrieren des angefallenen Niederschlags mit laborüblichen Methoden aus dem Filtrat erfolgen. Beispielsweise besteht eine gängige und geeignete Methode darin, das Lösungsmittel abzudestillieren, das Rohprodukt erneut in einem organischen Lösungsmittel aufzunehmen, die erhaltene Lösung mit Wasser mehrfach zu extrahieren, das Lösungsmittel erneut abzudestillieren und das erhaltene Produkt durch Umkristallisation aus einem geeigneten Lösungsmittel, wie z. B. aus Methanol umzukristallisieren. Es sind aber auch andere, dem Fachmann bekannte Aufarbeitungsvarianten möglich, wie z. B. solche, die eine chromatographische Aufreinigung mit einschließen.

Je nach Reaktionsbedingungen, wird das Reaktionsprodukt (III) als freie Base oder als Säureadditionssalz der Säuren HCl, HBr, HI, H₂SO₄ oder einer organischen Carbonsäure aus einem wasserhaltigen Lösungsmittelgemisch erhalten. In letzteren Fällen kann die Isolierung nach der Phasentrennung nach laborüblichen Methoden erfolgen.

Als geeignete organische Carbonsäuren können insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure. Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure verwendet werden.

Die Reduktion der Verbindungen der Formel (III) erfolgt unter Schutzgasatmosphäre, z. B. unter Stickstoffatmosphäre, in Gegenwart eines Hydridtransfer-Reagenzes. Geeignete Hydridtransfer-Reagenzien sind solche aus der Gruppe der Metallaluminiumhydride, vorzugsweise Lithiumaluminiumhydrid, Metall-Alkoxy-aliminiumhydride, wie z. B. Li-Triethoxyaluminiumhydrid, Metallborhydride, vorzugsweise NaBH₄, oder Boran, wobei zusätzlich die Gegenwart einer Lewissäure erforderlich ist, wie z. B. Bortrifluorid.

Die Reduktion wird vorzugsweise in einem polaren aprotischen und hydridinerten Lösungsmittel durchgeführt. Geeignet sind die gleichen, wie oben bereits genannt. Besonders geeignet sind z. B. Diethylether oder Tetrahydrofuran.

Zur Durchführung der Hydrierung wird eine Verbindung der Formel (III) in einem geeigneten Lösungsmittel gelöst und zu einer Lösung, die das Hydridtransfer-Reagenz in äquimolaren Mengen bzw. in geringem Überschuß enthält, unter Erwärmen zugegeben. Es ist aber auch möglich, die zu hydrierende Ausgangsverbindung vorzulegen und das Hydrierungsreagenz in entsprechender Menge auf geeignete Weise zuzugeben, so daß ein Reaktionsgemisch erhalten wird, worin das Edukt eine Konzentration von 10 bis 25 Gew.-% besitzt, bezogen auf das Lösungsmittel. Zur Beendigung der Reaktion wird das Reaktionsgemisch für mehrere Stunden unter Rückflußbedingungen gerührt. Die Reaktionslösung wird anschließend nach dem Fachmann bekannten Methoden aufbereitet, indem u. a. durch Zugabe eines Lösungsmittelgemisches, bestehend aus einem protonenliefernden und einem aprotischen Lösungsmittel, der Überschuß Hydridtransfer-Reagenz zersetzt und das Reaktionsprodukt freigesetzt wird. Als protonenliefernde Lösungsmittel sind z. B. Wasser oder Alkohole wie Ethanol oder Methanol geeignet. Als aprotische Lösungsmittel sind alle oben bereits genannten polaren aprotischen Lösungsmittel geeignet, insbesondere Tetrahydrofuran. Letzteres wird vorzugsweise eingesetzt, da es technisch als wasserfreies Produkt erhältlich ist.

Die Produktaufarbeitung kann nach der Phasentrennung nach laborüblichen Methoden erfolgen. Das erhaltene Rohprodukt kann durch Kristallisationsmethoden aufgearbeitet werden oder es wird zur Aufarbeitung beispielsweise in einem organischen nicht wassermischbaren Lösungsmittel aufgenommen und mit einem Überschuß einer anorganischen Säure, vorzugsweise Salzsäure, versetzt. Das auf diese Weise gebildete Salz kann anschließend kristallin abgetrennt werden.

Die weitere Umsetzung von N-Methyl-N-[(1S)-1-phenyl-2-((3S)-3-hydroxypyrrolidin-1-yl)-ethan]-amin oder dessen Dihydrochlorid mit einem geeigneten Diphenylessigsäurederivat, vorzugsweise dem Säurechlorid, zu dem gewünschten Endprodukt N-Methyl-N-1(1S)-1-phenyl-2-((3S)-3-hydroxypyrrolidin-1-yl)-ethyl]-2,2-diphenylacetamid (Formel VI, EMD 61753) erfolgt nach Methoden wie sie in DE-A1-40 34 785 und DE-A1-42 15 213 bzw. EP 0 569 802 A1 beschrieben sind.

Die im nachfolgenden gegebenen Beispiele werden zur Veranschaulichung der vorliegenden Erfindung gegeben, können aber nicht dazu dienen, die beanspruchte Erfindung auf diese zu beschränken, da verschiedene Variationen der Beispiele möglich sind und zu dem gewünschten Produkt N-Methyl-N-[(1S)-1-phenyl-2-((3S)-3-hydroxypyrrolidin-1-yl)-ethan]-amin [Formel (IV)] führen, das als Zwischenprodukt zur Herstellung von N-Methyl-N-[(1S)-1-phenyl-2-((3S)-3-hydroxypyrrolidin-1-yl)-ethyl]-2,2-diphenylacetamid verwendet werden kann.

### BEISPIELE

N-substituierte (2S)-2-Phenylglycin-N,N-[(3S)-3-hydroxy-tetramethylen-amide] der Formel III aus (2S)-2-Phenylglycinen der Formel I

### Beispiel 1 (Vergleich)

### (2S)-N-Formyl-2-phenylglycin-N,N-[(3S)-3-hydroxy-tetramethylenamid]

Aus (2S)-N-Formyl-2-phenylglycin (erhältlich aus (S)-(+)-alpha-Amino-phenylessigsäure und Acetanhydrid / Ameisensäure z.B. nach Huszthy, Peter: Oue, Masatoshi; Bradshaw, Jerald S.; Zhu, Cheng Y.; Wang, Tingmin; et al., J.Org.Chem., EN, 57 (20) [1992] 5383-5394)
und
(3S)-3-Hydroxypyrrolidin (erhältlich aus kommerziellem (S)-1-Benzyl-3-pyrrolidinol z.B. nach Bhat, Krishna L.; Flanagan, Denise M.; Joullie, Madeleine M., Synth.Commun., EN, 15 (7) [1985] 587-598 oder Naylor, Alan: Judd, Duncan B.; Scopes, David I. C.; Hayes, Ann G.; Birch, Philip J., J.Med.Chem., EN, 37 (14) [1994] 2138-2144):

Unter Stickstoffatmosphäre werden zu 9 g (2S)-N-Formyl-2-phenylglycin und 5,5 ml N-Methylmorpholin in 250 mL THF bei -15°C 4,8 ml Ethylchlorformiat in 10 mL Tetrahydrofuran unter Rühren zugesetzt und nach 10 min Wartezeit eine Lösung von 6,2 g (3S)-3-Hydroxypyrrolidin-Hydrochlorid und 7 mL Triethylamin in 50 mL Dimethylformamid. Nach 18 Stunden Rühren wird der angefallene Niederschlag abgetrennt und entstandenes (2S)-N-Formyl-2-phenylglycin-N,N-[(3S)-3-hydroxy-tetramethylenamid mit laborüblichen Methoden durch Aufkonzentrieren, und anschließende chromatographische Reinigung aus dem Filtrat isoliert.
1H-NMR: D₆-DMSO; 3,0-3,8 (m), 4,25 (d), 5,0 (s,br), 5,7 (dd), 7,4 (ArH), 8,0 (ArH), 8,8 (CHO):
MS-FAB: (M+1)⁺ 221, 205;
Kristalle Fp.: 97-101°C;
[α]_{D}²⁰ = +208, c=1 in Methanol.

### Beispiel 2 (Vergleich)

### (2S)-N-Carboxybenzyl-2-phenylglycin-N,N-[(3S)-3-hydroxytetramethylenamid]

Aus (2S)-N-Carboxybenzyl-2-phenylglycin (aus (S)-(+)-alpha-Aminophenylessigsäure und Chlorkohlensäurebenzylester z.B. nach Jones, Raymond CF; Turner, lan; Howard, Kevin J., Tetrahedron Lett., 34 (39) [1993] 6329-6332)
und
(3S)-3-Hydroxypyrrolidin (erhältlich aus kommerziellem (S)-1-Benzyl-3-pyrrolidinol z.B. nach Bhat, Krishna L.; Flanagan, Denise M.; Joullie, Madeleine M., Synth.Commun., EN, 15 (7) [1985] 587-598 oder Naylor, Alan; Judd, Duncan B.; Scopes, David I. C.; Hayes, Ann G.; Birch, Philip J., J.Med.Chem., EN, 37 (14) [1994] 2138-2144):
Unter Stickstoffatmosphäre werden 14,3 g (2S)-N-Carboxy-benzyl-2-phenylglycin in 100 ml Tetrahydrofuran in der Kälte mit 5,5 ml 4-Methylmorpholin und einer Lösung aus 4,8 ml Ethylchlorformiat und 10 ml Tetrahydrofuran versetzt und dann 30 min gerührt. Dann wird eine Lösung aus 4.36 g (3S)-3-Hydroxypyrrolidin und 10 ml Tetrahydrofuran zugeben. Nach 18 Stunden Rühren wird der angefallene Niederschlag abgetrennt und das gebildete (2S)-N-Carboxybenzyl-2-phenylglycin-N,N-[(3S)-3-hydroxy-tetramethylenamid mit laborüblichen Methoden durch Aufkonzentrieren, Aufnehmen in einem organischen Solvens, Waschen
mit einer wäßrigen Phase, erneutes Aufkonzentrieren und Kristallisation aus dem Filtrat isoliert.
1H-NMR: D₆-DMSO+TFA; 5,1 (s), PhCH₂R;
FAB-MS: 355 (M+1)⁺, 311, 196, 176;
Konsistenz: Öl;
[α]_{D}²⁰ = + 108, c=1 in Methanol.

### Beispiel 3

### (2S)-N-Carboxyethyl-2-phenylglycin-N,N-[(3S)-3-hydroxy-tetramethylenamid]

### 3.a)

Aus (2S)-N-Carboxyethyl-2-phenylglycin (aus (S)-(+)-alpha-Amino-phenylessigsäure und Chlorkohlensäureethylester z.B. nach Bodurow, C. C.; Boyer, B. D.; Brennan, J.; Bunnell, C. A.; Burks, J. E.; et al., Tetrahedron Lett., EN, 30 (18) [1989] 2321-2324)
und
(3S)-3-Hydroxypyrrolidin (erhältlich aus kommerziellem (S)-1-Benzyl-3-pyrrolidinol z.B. nach Bhat, Krishna L.; Flanagan, Denise M.; Joullie, Madeleine M., Synth.Commun., EN, 15 (7) [1985] 587-598 oder Naylor, Alan; Judd, Duncan B.; Scopes, David I. C.; Hayes, Ann G.; Birch, Philip J., J.Med.Chem., EN, 37 (14) [1994] 2138-2144)

Unter Stickstoffatmosphäre werden 16,7 g (2S)-N-Carboxyethyl-2-phenylglycin in 100 ml Tetrahydrofuran in der Kälte mit 8,3 ml 4-Methylmorpholin und einer Lösung aus 7,1 ml Ethylchlorformiat und 20 ml Tetrahydrofuran versetzt und dann 60 min gerührt. Dann wird eine Lösung aus 6,5 g (3S)-3-Hydroxypyrrolidin und 30 ml Tetrahydrofuran zugeben. Nach 18 Stunden Rühren wird der angefallene Niederschlag abgetrennt und das (2S)-N-Carboxyethyl-2-phenylglycin-N,N-[(3S)-3-hydroxy-tetramethylenamid mit laborüblichen Methoden durch Aufkonzentrieren, Aufnehmen in einem organischen Solvens, Waschen mit einer wäßrigen Phase, erneutes Aufkonzentrieren und Kristallisation aus dem Filtrat isoliert.

### 3.b)

Aus (2S)-N-Carboxyethyl-2-phenylglycin (s.o.) und (3S)-3-Hydroxypyrrolidin-Hydrochlorid (kommerziell erhältlich): Zu 11 g Ethylchlorformiat in 100 ml THF werden bei ca - 10 °C eine Mischung von 24 g (2S)-N-Carboxyethyl-2-phenylglycin mit 10 g Methylmorpholin in 100 ml THF dosiert. Es folgt nach einer Nachrührphase eine Mischung von 12 g (3S)-3-Hydroxypyrrolidin-Hydrochlorid in 10 ml VE-Wasser sowie eine Mischung von 10 g Methylmorpholin in 20 ml THF. Nach mehrstündigem Nachrühren und Phasentrennung wird das (2S)-N-Carboxyethyl-2-phenylglycin-N,N-[(3S)-3-hydroxy-tetramethylenamid mit laborüblichen Methoden durch Aufkonzentrieren, Aufnehmen in einem organischen Solvens, Waschen mit einer wäßrigen Phase, erneutes Aufkonzentrieren und Kristallisation isoliert.

Die analytischen Daten zu den Varianten 3a und 3b entsprechen sich:
1H-NMR: D₆-DMSO; 1,2 (t), 3 - 3,8 (m, br), 4,05 (q), 4,25 (s, br), 7,25-7,45 (m);
MS: 293 (M+1)⁺, 247, 178, 106;
Kristalle Fp.: 124-126°C;
[α]_{D}²⁰ = + 137 C = 1 in Methanol.

### N-Methyl-N-[(1S)-1-phenyl-2-((3S)-3-hydroxypyrrolidin-1-yl)-ethan]-amin der Formel IV

### Beispiel 4

### N-Methyl-N-[(1S)-1-phenyl-2-((3S)-3-hydroxypyrrolidin-1-yl)-ethan]-amin =

1-[(3S)-3-Hydroxypyrrolidin-1-yl]-(2S)-2-methyl-amino-2-phenylethan Unter Stickstoff werden 2200 ml einer 1,08 molaren Lithiumaluminiumhydrid-Tetrahydrofuran-Lösung leicht erwärmt und unter Rühren eine Lösung aus 264 g (2S)-N-Carboxyethyl-2-phenylglycin-N,N-[(3S)-3-hydroxy-tetramethylenamid] und 1400 ml Tetrahydrofuran zudosiert. Nach dem Dosierungsende wird 3 Stunden refluxiert und die erkaltete Reaktionslösung mittels einer Wasser/Tetrahydrofuran-Mischung hydrolysiert. Nach Natriumcarbonat-Behandlung und Abtrennung anorganische Bestandteile wird das Produkt mit laborüblichen Methoden aus dem Filtrat isoliert. Das ölige Rohprodukt bildet nach Aufreinigung mittels Kristallisation oder Chromatographie einen Feststoff.
1H-NMR: D₆-DMSO; 2,1-3,1(m), 3,6(dd), 4,3(m), 7,15-7,35 (m);
MS: 220 (M⁺), 205, 120, 100, 91;
Aussehen: gelbliches Öl, welches chargenabh. durchkristallisiert;
[α]_{D}²⁰ = + 66,8; c = 0,0938 g in 10 ml Methanol.

### Beispiel 5

### N-Methyl-N-[(1S)-1-phenyl-2-((3S)-3-hydroxypyrrolidin-1-yl)-ethan]amin-dihydrochlorid =

### 1-[(3S)-3-Hydroxypyrrolidin-1-yl]-(2S)-2-methyl-amino-2-phenylethan Dihydrochlorid

Unter Stickstoff werden 2200 ml einer 1,08 molaren Lithiumaluminiumhydrid-Tetrahydrofuran-Lösung leicht erwärmt und unter Rühren eine Lösung aus 264 g (2S)-N-Carboxyethyl-2-phenylglycin-N,N-[(3S)-3-hydroxy-tetramethylenamid] und 1400 mL Tetrahydrofuran zudosiert. Nach dem Dosierungsende wird noch 3 Stunden refluxiert, dann abgekühlt und die Reaktionslösung mittels einer Mischung aus 80 ml Wasser und 400 ml Tetrahydrofuran hydrolysiert. Nach Natriumcarbonat-Behandlung und Abtrennung anorganische Bestandteile wird das Produkt mit laborüblichen Methoden aus dem Filtrat isoliert. Das ölige Rohprodukt wird in einem organischen, nicht wassermischbarem Lösungsmittel aufgenommen und mit einem Überschuß Salzsäure versetzt. Das kristalline Produkt wird isoliert und getrocknet.
1H-NMR: D₆-DMSO; 3,4(m), 3,8(m), 4,2(m), 4,4(m), 4,9(m), 7,5 u. 7,8 (ArH);
Schmelzpunkt: 240-242°C;
[α]_{D}²⁰ = -22.4; c=1 in Wasser.

## Patentansprüche

1. Verfahren zur wahlweisen Herstellung von N-Methyl-N-[(1S)-1-phenyl-2-((3S)-3-hydroxypyrrolidin-1-yl)ethyl]-2,2-diphenylacetamid oder N-Methyl-N-[(1R)-1-phenyl-2-((3R)-3-hydroxypyrrolidin-1-yl)ethyl]-2,2-diphenylacetamid, **dadurch gekennzeichnet, daß**
a) ein N-substituiertes Phenylglycinderivat der Formel I 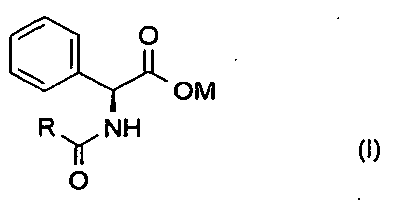 worin
R OR¹ oder SR¹,
R¹ A, Aryl, Heteroaryl, Si(R³)₃ oder COR³,
R³ H, A, Aryl oder Heteroaryl,
A geradkettiger oder verzweigter Alkylrest mit 1 bis 6 C-Atomen,
M H oder ein Kation aus der Gruppe Alkali-, Erdalkali-Ammonium- oder Alkylammonium
bedeuten,
mit einer Verbindung der Formel II 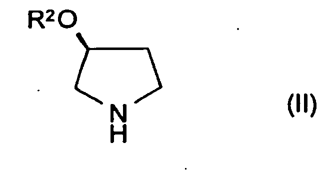 worin
R² H, A, Aryl, Heteroaryl, Si(R³)₃ oder COR³
und
R³ H, A, Aryl oder Heteroaryl
bedeuten
oder mit einem Säureadditionssalz der Verbindung der Formel (II), der Säuren HCl, HBr, HI, H₂SO₄, H₃PO₄ oder einer organischen Carbonsäure, zu einer Verbindung der Formel III 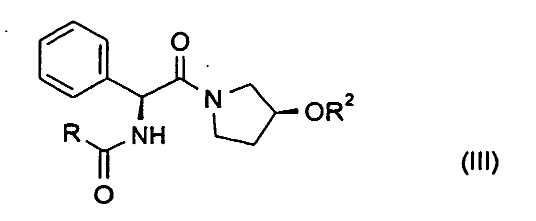 worin R und R² die oben gegebenen Bedeutungen haben,
umgesetzt wird,
b) anschließend durch Reduktion in eine Verbindung der Formel (IV) 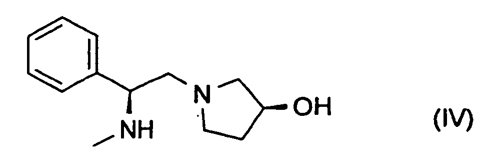 umgesetzt wird, die gegebenenfalls in ein entsprechendes Säureadditionssalze der Säuren HCl, HBr, HI, H₂SO₄, H₃PO₄ oder in ein Salz einer organischen Carbonsäure umgewandelt wird, und
c) die so erhaltene Verbindung der Formel (IV) mit einer aktivierten Carbonsäure der Formel (V) 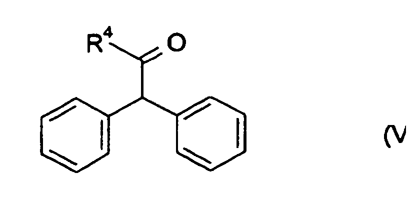 worin
R⁴ F, Cl, Br, I, OA oder O-CO-A,
bedeutet,
zu der Verbindung der Formel (VI) 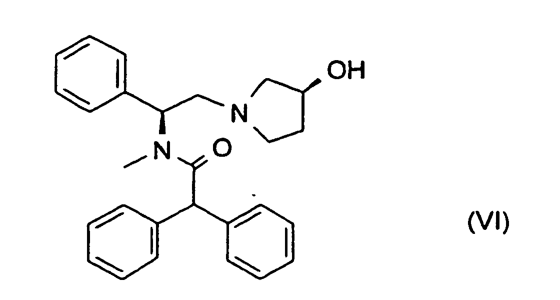 umgesetzt wird,
die gegebenenfalls mit einer anorganischen Säure aus der Gruppe HCl, HBr, HI, Schwefelsäure, Sulfaminsäure, Salpetersäure, Phosphorsäure, Orthophosphorsäure, oder mit einer organischen Säure in das zugehörige Säureadditionssalz überführt wird,
wobei in Stufe a) Edukte in Abhängigkeit vom als Endprodukt gewünschten Enantiomeren eingesetzt werden.

2. Verfahren gemäß Anspruch 1, **dadurch gekennzeichnet, daß** Verbindungen der Formel (I) verwendet werden, in denen R die Bedeutung OR¹ mit R¹ A, Aryl, Heteroaryl, Si(R³)₃ oder COR³ und R³ H, A, Aryl oder Heteroalkyl besitzt.

3. Verfahren gemäß der Ansprüche 1 und/oder 5, **dadurch gekennzeichnet, daß** die Umsetzung der Verbindungen (I) und (II) in einem aprotischen, vorzugsweise polaren aprotischen Lösungsmittel bei einer Temperatur von 0 bis 50 °C, vorzugsweise bei Temperaturen zwischen 20 und 30 °C, erfolgt.

4. Verfahren gemäß einem oder mehreren der Ansprüche 1, 5 und 6, **dadurch gekennzeichnet, daß** die Umsetzung der Verbindungen (I) und (II) in einem Lösungsmittel aus der Gruppe Dieethylether, Petrolether, Aceton, Nitrobenzol, Dimethylformamid, Dimethylsulfoxid, Tetrahydrofuran durchgeführt wird, wobei die Edukte in dem Lösungsmittel in einer Konzentration von 10 bis 30 % vorliegen.

5. Verfahren gemäß einem oder mehreren der Ansprüche 1, 5 bis 7, **dadurch gekennzeichnet, daß** die Umsetzung der Verbindungen (I) und (II) in Gegenwart eines Hilfsreagenzes aus der Gruppe Phosphoroxytrichlorid, Phosphorhalogenide der Wertigkeit III und V, Phosgen, Dicyclohexylcarbodiimid, Tributylammoniumsalz des Pyridins, Phenyldichlorphosphat, 2-Chlor-1,2,3-trinitrobenzol, Phosphorsäureester, Chlorsulfonylisocyanat, CH₃SO₂Cl-(C₂H₅)₃N, (C₆H₅)₃P-CCl₄-(C₂H₅)₃N, N,N'-Carbonyldiimidazol, N-(Alkylcarbonyl)-imidazole, Acetanhydrid, Essigsäurechlorid und Chlorameisensäureethylester sowie einer organischen oder anorganischen Base erfolgt.

6. Verfahren gemäß einem oder mehreren der Ansprüche 1, 5 bis 8, **dadurch gekennzeichnet, daß** die Umsetzung der Verbindungen (I) und (II) in Gegenwart einer Base aus der Gruppe Triethylamin, Natriumcarbonat, Kaliumcarbonat, Kalziumcarbonat, NaOH, KOH erfolgt.

7. Verfahren gemäß einem oder mehreren der Ansprüche 1, 5 bis 9 **dadurch gekennzeichnet, daß** die Reduktion der Verbindungen der Formel (III) in Gegenwart eines Hydridtransfer-Reagenzes aus der Gruppe der Metallaluminiumhydride, vorzugsweise Lithiumaluminiumhydrid, der Metall-Alkoxy-aliminiumhydride, vorzugsweise Li-Triethoxyaluminiumhydrid, der Metallborhydride, vorzugsweise NaBH₄, oder Boran, sowie gegebenenfalls in Gegenwart einer Lewissäure, wie Bortrifluorid, in einem polaren aprotischen Lösungsmittel aus der Gruppe Dieethylether, Petrolether, Aceton, Nitrobenzol, Dimethylformamid, Dimethylsulfoxid, Tetrahydrofuran erfolgt.

8. Verfahren gemäß den Ansprüchen 1 und/oder 10, **dadurch gekennzeichnet, daß** eine Verbindung der Formel (III) als Edukt in einem Lösungsmittel in einer Konzentration von 10 bis 25 % gelöst wird und das Hydrierungsprodukt durch Zugabe eines protonenliefernden Lösungsmittels im Gemisch mit einem aprotischen Lösungsmittel freigesetzt wird.

## Claims

1. Process for the preparation of either N-methyl-N-[(1S)-1-phenyl-2-((3S)-3-hydroxypyrrolidin-1-yl)ethyl]-2,2-diphenylacetamide or N-methyl-N-[(1R)-1-phenyl-2-((3R)-3-hydroxypyrrolidin-1-yl)ethyl]-2,2-diphenylacetamide, **characterised in that**
a) an N-substituted phenylglycine derivative of the formula I 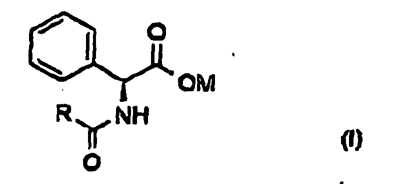 in which
R denotes OR¹ or SR¹,
R¹ denotes A, aryl, heteroaryl, Si(R³)₃ or COR³,
R³ denotes H, A, aryl or heteroaryl,
A denotes a straight-chain or branched alkyl radical having 1 to 6 C atoms,
M denotes H or a cation from the group alkali, alkaline earth ammonium or alkylammonium,
is reacted with a compound of the formula II 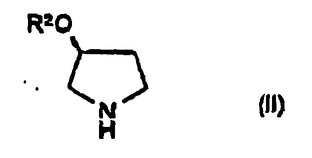 in which
R² denotes H, A, aryl, heteroaryl, Si(R³)₃ or COR³ and
R³ denotes H, A, aryl or heteroaryl
or with an acid-addition salt of the compound of the formula (II) of the acids HCI, HBr, HI, H₂SO₄, H₃PO₄ or an organic carboxylic acid, to give a compound of the formula III 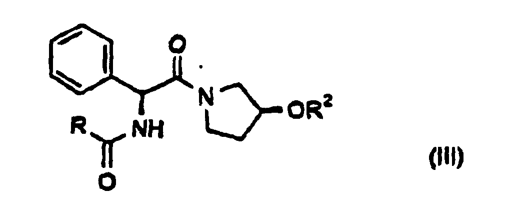 in which R and R² have the meanings given above,
b) subsequently converted by reduction into a compound of the formula (IV) 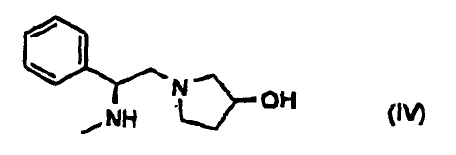 which is, if desired, converted into a corresponding acid-addition salt of the acids HCl, HBr, HI, H₂SO₄, H₃PO₄ or into a salt of an organic carboxylic acid, and
c) the resultant compound of the formula (IV) is reacted with an activated carboxylic acid of the formula (V) 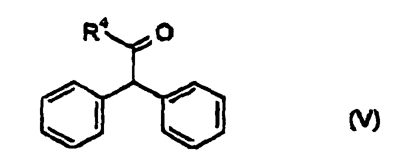
in which
R⁴ denotes F, Cl, Br, I, OA or O-CO-A,
to give the compound of the formula (VI) 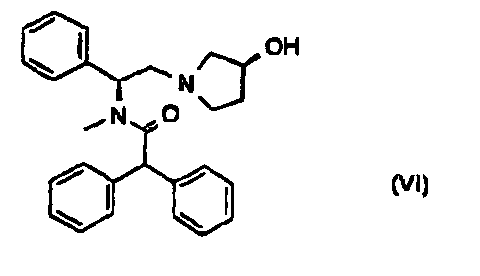 which is, if desired, converted into the associated acid-addition salt with an inorganic acid from the group HCl, HBr, HI, sulfuric acid, sulfamic acid, nitric acid, phosphoric acid, orthophosphoric acid, or with an organic acid,
where, in step a), starting materials depending on the enantiomer desired as end product are employed.

2. Process according to Claim 1, **characterised in that** use is made of compounds of the formula (I) in which R has the meaning OR¹, where R¹ is A, aryl, heteroaryl, Si(R³)₃ or COR³ and R³ is H, A, aryl or heteroalkyl.

3. Process according to Claims 1 and/or 5, **characterised in that** the reaction of the compounds (I) and (II) is carried out in an aprotic, preferably polar aprotic solvent at a temperature of 0 to 50°C, preferably at temperatures between 20 and 30°C.

4. Process according to one or more of Claims 1, 5 and 6, **characterised in that** the reaction of the compounds (I) and (II) is carried out in a solvent from the group diethyl ether, petroleum ether, acetone, nitrobenzene, dimethylformamide, dimethyl sulfoxide, tetrahydrofuran, the starting materials being present in the solvent In a concentration of 10 to 30%.

5. Process according to one or more of Claims 1, 5 to 7, **characterised in that** the reaction of the compounds (I) and (II) is carried out in the presence of an auxiliary reagent from the group phosphorus oxytrichloride, phosphorus halides having the valency III and V, phosgene, dicyclohexylcarbodiimide, the tributylammonium salt of pyridine, phenyl dichlorophosphate, 2-chloro-1,2,3-trinitrobenzene, phosphoric acid esters, chlorosulfonyl isocyanate, CH₃SO₂Cl-(C₂H₅)₃N, (C₆H₅)₃P-CCl₄-(C₂H₅)₃N, N,N'-carbonyldilmidazole, N-(alkylcarbonyl)imidazoles, acetic anhydride, acetyl chloride and ethyl chloroformate, and of an organic or inorganic base.

6. Process according to one or more of Claims 1, 5 to 8, **characterised in that** the reaction of the compounds (I) and (II) is carried out in the presence of a base from the group triethylamine, sodium carbonate, potassium carbonate, calcium carbonate, NaOH, KOH.

7. Process according to one or more of Claims 1, 5 to 9, **characterised in that** the reduction of the compounds of the formula (III) is carried out in the presence of a hydride transfer reagent from the group of the metal aluminium hydrides, preferably lithium aluminium hydride, the metal alkoxyaluminlum hydrides, preferably Li triethoxyaluminium hydride, the metal borohydrides, preferably NaBH₄, or borane, and if desired in the presence of a Lewis acid, such as boron trifluoride, in a polar aprotic solvent from the group diethyl ether, petroleum ether, acetone, nitrobenzene, dimethylformamide, dimethyl sulfoxide, tetrahydrofuran.

8. Process according to Claims 1 and/or 10, **characterised in that** a compound of the formula (III) as starting material is dissolved in a solvent in a concentration of 10 to 25%, and the hydrogenation product is liberated by addition of a proton-supplying solvent mixed with an aprotic solvent.

## Revendications

1. Procédé pour la préparation de soit N-méthyl-N-[(1S)-1-phényl-2-((3S)-3-hydroxypyrrolidine-1-yl)éthyl]-2,2-diphénylacétamide, soit N-méthyl-N-[(1R)-1-phényl-2-((3R)-3-hydroxypyrrolidine-1-yl)éthyl]-2,2-diphényl-acétamide, **caractérisé en ce que**
a) un dérivé de phénylglycine N-substitué de la formule I 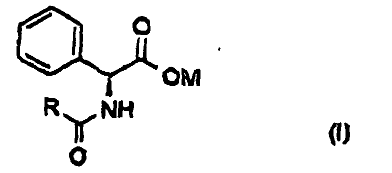 dans laquelle
R représente OR¹ ou SR¹,
R¹ représente A, aryle, hétéroaryle, Si(R³)₃ ou COR³,
R³ représente H, A, aryle ou hétéroaryle,
A représente un radical alkyle en chaîne droite ou ramifié comportant de 1 à 6 atomes de C,
M représente H où un cation pris parmi le groupe alcali, ammonium alcalinoterreux ou alkylammonium,
est amené à réagir avec un composé de la formule II 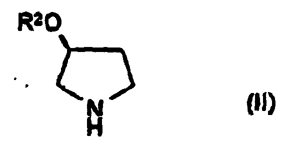 dans laquelle
R² représente H, A, aryle, hétéroaryle, Si(R³)₃ ou COR³ et
R³ représente H, A, aryle ou hétéroaryle
ou avec un sel par addition d'acide du composé de la formule (II) des acides HCl, HBr, HI, H₂SO₄, H₃PO₄ ou d'un acide carboxylique organique, pour donner un composé de la formule III 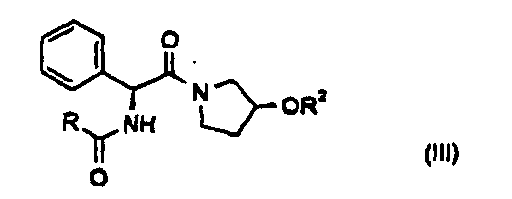 dans laquelle R et R² présentent les significations données ci-avant,
b) est ensuite converti par réduction selon un composé de la formule (IV) 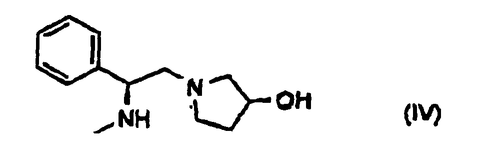 qui est, si on le souhaite, converti selon un sel par addition d'acide correspondant des acides HCl, HBr, HI, H₂SO₄, H₃PO₄ ou selon un sel d'un acide carboxylique organique, et
c) le composé résultant de la formule (IV) est amené à réagir avec un acide carboxylique activé de la formule (V) 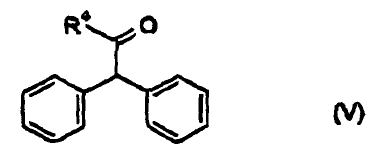
dans laquelle
R⁴ représente F, Cl, Br, I, OA ou O-CO-A,
pour donner le composé de la formule (VI) 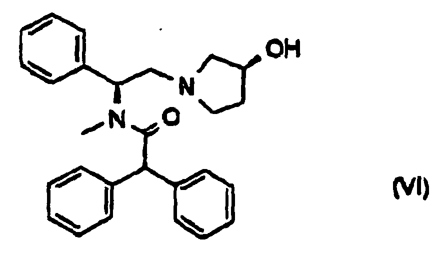 qui est, si on le souhaite, converti selon le sel par addition d'acide associé avec un acide inorganique pris parmi le groupe HCI, HBr, HI, acide sulfurique, acide sulfamique, acide nitrique, acide phosphorique, acide orthophosphorique, ou avec un acide organique,
où, au niveau de l'étape a), des matériaux de départ en fonction de l'énantiomère souhaité en tant que produit final sont utilisés.

2. Procédé selon la revendication 1, **caractérisé en ce que** l'on utilise des composés de la formule (I) dans laquelle R présente la signification OR¹, où R¹ est A, aryle, hétéroaryle, Si(R³)₃ ou COR³ et R³ est H, A, aryle ou hétéroalkyle.

3. Procédé selon les revendications 1 et/ou 5, **caractérisé en ce que** la réaction des composés (I) et (II) est mise en oeuvre dans un solvant aprotique, de préférence aprotique polaire à une température de 0 à 50°C, de préférence à des températures entre 20 et 30°C.

4. Procédé selon une ou plusieurs des revendications 1, 5 et 6, **caractérisé en ce que** la réaction des composés (I) et (II) est mise en oeuvre dans un solvant pris parmi le groupe éther diéthylique, éther de pétrole, acétone, nitrobenzène, diméthylformamide, sulfoxyde diméthylique, tétrahydrofurane, les matériaux de départ étant présents dans le solvant selon une concentration de 10 à 30%.

5. Procédé selon une ou plusieurs des revendications 1, 5 à 7, **caractérisé en ce que** la réaction des composés (I) et (II) est mise en oeuvre en présence d'un réactif auxiliaire pris parmi le groupe oxytrichlorure de phosphore, halogénures de phosphore présentant la valence III et V, phosgène, dicyclohexylcarbodiimide, le sel tributylammonium de pyridine, dichlorophosphate de phényle, 2-chtoro-1,2,3-trinitrobenzène, esters d'acide phosphorique, isocyanate de chlorosulfonyle, CH₃SO₂Cl-(C₂H₅)₃N, (C₆H₅)₃P-CCl₄-(C₂H₅)₃N, N,N'-carbonyldiimidazole, N-(alkylcarbonyl)imidazoles, anhydride acétique, chlorure d'acétyle et chicroformate d'éthyle, et d'une base organique ou inorganique.

6. Procédé selon une ou plusieurs des revendications 1, 5 à 8, **caractérisé en ce que** la réaction des composés (I) et (II) est mise en oeuvre en présence d'une base prise parmi le groupe triéthylamine, carbonate de sodium, carbonate de potassium, carbonate de calcium, NaOH, KOH,

7. Procédé selon une ou plusieurs des revendications 1, 5 à 9, **caractérisé en ce que** la réduction des composés de la formule (III) est mise en oeuvre en présence d'un réactif de transfert d'hydrure pris parmi le groupe des hydrures de métal-aluminium, de préférence hydrure de lithium-aluminium, des hydrures de métal-alkoxyaluminium, de préférence hydrure de Li-triéthoxyaluminium, des borohydrures de métal, de préférence NaBH₄, ou borane, et si on le souhaite en présence d'un acide de Lewis, tel que trifluorure de bore, dans un solvant aprotique polaire pris parmi le groupe éther diéthylique, éther de pétrole, acétone, nitrobenzène, diméthylformamide, sulfoxyde diméthylique, tétrahydrofurane.

8. Procédé selon les revendications 1 et/ou 10, **caractérisé en ce qu'**un composé de la formule (III) en tant que matériau de départ est dissout dans un solvant selon une concentration de 10 à 25%, et le produit d'hydrogénation est libéré par addition d'un solvant de fourniture de protons mélangé avec un solvant aprotique.
